# EUROPEAN PATENT APPLICATION

(11) **EP 4 722 733 A1**
(43) Date of publication of application: **08.04.2026**
(21) Application number: 23940561.6
(22) Date of filing: 05.06.2023
(51) Int. Cl.: G01N 35/08, G01N 37/00

(54) **SAMPLE SOLUTION SEPARATION DEVICE, SAMPLE SOLUTION SEPARATION SYSTEM, AND SAMPLE SOLUTION SEPARATION METHOD**

(71) Applicant: HITACHI HIGH-TECH CORPORATION, Tokyo 105-6409 (JP)
(72) Inventor: NAKAGAWA Tatsuo, Tokyo 100-8280 (JP); KAMURA Yoshio, Tokyo 100-8280 (JP)
(74) Representative: Strehl & Partner mbB
(86) International application number: PCT/JP2023/020780
(87) International publication number: WO 2024/252453

(57) **Abstract**

Provided is a sample solution separation device affording minimal dead volume. A sample solution separation device 101 includes a plurality of microchambers 105; a flow path 104 connecting the plurality of microchambers 105; a first opening 102 constituting an inlet through which the sample solution is introduced into the flow path 104; a valve 103 provided between the first opening 102 and the plurality of microchambers 105; a second opening 107 provided on an opposite side to the first opening such that the plurality of microchambers are sandwiched between the first and second openings, the second opening being an inlet to a flow path of a separation liquid for separation between the plurality of microchambers 105 in which the sample solution is stored; and a solid phase 106 provided between the second opening 107 and the plurality of microchambers 105, wherein the solid phase 106 has air permeability, water repellency with respect to the sample solution, and permeability with respect to the separation liquid.

## Description

### Technical Field

The present invention relates to a sample solution separation device for separating a sample solution, a sample solution separation system, and a sample solution separation method.

### Background Art

Digital PCR (Polymerase Chain Reaction) is a technique for detecting nucleic acids with high sensitivity, and when compared with conventional real-time quantitative PCR, is capable of detecting low-frequency genetic mutations.

In digital PCR, a sample solution is divided into minute volumes, and then PCR is performed on the divided solutions and the DNA to be measured is amplified and measured. As a method for putting a sample solution into a minute container, PTL 1 discloses a method in which a port is sealed and the interior of the container is placed under a vacuum to draw the sample solution into the container. In addition, NPL 1 discloses a method in which the interior of a container is degassed, a valve on an outlet side is closed, and a valve on an inlet side is opened to introduce a solution. PTL 2 discloses a configuration in which a solution is discharged without waste by using a gas-liquid separation filter.

### Citation List

### Patent Literature

PTL 1: U.S. Patent No. 11305276
PTL 2: JP 4888773 A

### Non Patent Literature

NPL 1: Micromachines 2020, 11, 1025; doi:10.3390/mi11121025

### Summary of Invention

### Technical Problem

In digital PCR, a sample solution containing the DNA to be detected is separated into multiple sections, PCR is performed on each of the sections, and the type of DNA present in each of the sections is determined. Digital PCR is characterized in that the target DNA can be measured with high sensitivity by performing PCR after performing separation in each section. For example, a liquid biopsy for detecting cell-free DNA (cfDNA) present in blood in trace amounts is one example of a suitable application. Cell-free DNA may include ctDNA (circulating tumor DNA), which is DNA derived from a tumor, and by measuring the types and proportions of mutations in ctDNA, applications in the diagnosis of cancer, in treatment selection, and in monitoring therapeutic effects are to be expected.

Because cfDNA and ctDNA are present in blood in trace amounts, it is necessary to perform measurement with high sensitivity. In digital PCR, a sample solution is separated into a plurality of micro-volume chambers and measured. At such time, not all of the sample solution can be used, and the sample solution cannot be separated into micro-volume chambers and measured, that is, dead volume occurs. In order to perform the measurement with high sensitivity, it is necessary to measure the collected sample solution as much as possible. That is, it is necessary to reduce the dead volume of the sample solution.

The present invention was conceived of in view of such circumstances, and an object of the present invention is to provide a sample solution separation device, a sample solution separation system, and a sample solution separation method that enable a reduction in dead volume of a sample solution.

### Solution to Problem

In order to solve the above problems, the sample solution separation device of the present invention is a sample solution separation device that separates a sample solution and that includes a plurality of microchambers; a flow path connecting the plurality of microchambers; a first opening constituting an inlet through which the sample solution is introduced into the flow path; a valve provided between the first opening and the plurality of microchambers; a second opening provided on an opposite side to the first opening such that the plurality of microchambers are sandwiched between the first and second openings, the second opening being an inlet to a flow path of a separation liquid for separation between the plurality of microchambers in which the sample solution is stored; and a solid phase provided between the second opening and the plurality of microchambers, wherein the solid phase has air permeability, water repellency with respect to the sample solution, and permeability with respect to the separation liquid.

Furthermore, the sample solution separation system of the present invention includes a sample solution separation device that separates a sample solution and that includes a plurality of microchambers, a flow path connecting the plurality of microchambers, a first opening constituting an inlet through which the sample solution is introduced into the flow path, a valve provided between the first opening and the plurality of microchambers, a second opening provided on an opposite side to the first opening such that the plurality of microchambers are sandwiched between the first and second openings, the second opening being an inlet to a flow path of a separation liquid for separation between the plurality of microchambers in which the sample solution is stored, and a solid phase provided between the second opening and the plurality of microchambers, the solid phase having air permeability, water repellency with respect to the sample solution, and permeability with respect to the separation liquid, the sample solution separation system further including a pump that degasses the air in the plurality of microchambers and the flow path via the solid phase from the second opening; valve control means for opening the valve; and separation liquid introduction means for introducing the separation liquid from the second opening.

Furthermore, the sample solution separation method of the present invention includes preparing a sample solution separation device for separating a sample solution and that includes a plurality of microchambers, a flow path connecting the plurality of microchambers, a first opening constituting an inlet through which the sample solution is introduced into the flow path, a valve provided between the first opening and the plurality of microchambers, a second opening provided on an opposite side to the first opening such that the plurality of microchambers are sandwiched between the first and second openings, the second opening being an inlet to a flow path of a separation liquid for separation between the plurality of microchambers in which the sample solution is stored, and a solid phase provided between the second opening and the plurality of microchambers, the solid phase having air permeability, water repellency with respect to the sample solution, and permeability with respect to the separation liquid; degassing the air in the plurality of microchambers and the flow path via the solid phase; opening the valve and introducing the sample solution from the first opening to the plurality of microchambers and the flow path; and introducing the separation liquid from the second opening to the flow path via the solid phase.

### Advantageous Effects of Invention

With the sample solution separation device, the sample solution separation system, and the sample solution separation method of the present invention, it is possible to reduce dead volume of a sample solution when the sample solution is to be separated into a plurality of microchambers.

Other problems and novel features will become apparent from the description of the present specification and the accompanying drawings.

### Brief Description of Drawings

[FIG. 1] FIG. 1 is a configuration diagram of a sample solution separation device 101 according to a first embodiment.
[FIG. 2A] FIG. 2A is a diagram showing how a sample solution 201 and a separation liquid 202 are to be introduced into a sample solution separation device 101 according to the first embodiment.
[FIG. 2B] FIG. 2B is a diagram showing how the sample solution 201 and the separation liquid 202 are to be introduced into the sample solution separation device 101 according to the first embodiment.
[FIG. 2C] FIG. 2C is a diagram showing how the sample solution 201 and the separation liquid 202 are to be introduced into the sample solution separation device 101 according to the first embodiment.
[FIG. 2D] FIG. 2D is a diagram showing how the sample solution 201 and the separation liquid 202 are to be introduced into the sample solution separation device 101 according to the first embodiment.
[FIG. 3] FIG. 3 is a flowchart for when the sample solution 201 and the separation liquid 202 are introduced into the sample solution separation device 101 according to the first embodiment, and when the sample solution 201 is separated into microchambers 105.
[FIG. 4] FIG. 4 is a configuration diagram of a sample solution separation system 400 in which the sample solution 201 is separated and introduced into the sample solution separation device 101 according to the first embodiment.
[FIG. 5] FIG. 5 is a configuration diagram of a sample solution separation device 501 according to a second embodiment.
[FIG. 6A] FIG. 6A is a top view of a sample solution separation device 601 according to a third embodiment.
[FIG. 6B] FIG. 6B is a lateral view of the sample solution separation device 601 according to the third embodiment.
[FIG. 7] FIG. 7 is a configuration diagram of a sample solution separation system 700 that separates and introduces the sample solution 201 into the sample solution separation device 601 according to the third embodiment.
[FIG. 8] FIG. 8 is a configuration diagram of a sample solution separation device 801 according to a fourth embodiment.
[FIG. 9] FIG. 9 is a configuration diagram of a digital PCR system 900 according to a fifth embodiment.
[FIG. 10] FIG. 10 is an operation flowchart of the digital PCR system 900 according to the fifth embodiment.

### Description of Embodiments

In the following description, when it is necessary for convenience, the description will be divided into a plurality of sections or embodiments, but unless otherwise specified, the sections or embodiments are not unrelated to each other, with one section or embodiment being related to some or all modifications, details, supplementary explanations, and so forth of another section or embodiment. Furthermore, in the following description, when referring to numbers of elements and the like (including numbers, numerical values, amounts, ranges, and the like), the numbers of elements are not limited to specific numbers, and unless otherwise stated and unless clearly limited in principle to specific numbers, the number of elements may be equal to or greater than, or equal to or less than, the specific numbers.

Furthermore, in the following embodiments, it is understood that the constituent elements (including element steps and the like) are not necessarily essential unless otherwise specified or considered to be obviously essential in principle. Similarly, in the following embodiments, when reference is made to the shapes, positional relationships, and the like of the components and the like, the shapes and the like substantially approximate or similar to the shapes and the like are included unless otherwise specified or unless clearly considered in principle. The same applies to the above numerical values and ranges.

Note that, in all the drawings to illustrate the embodiments, the same members are generally assigned the same reference signs, and repetitive descriptions thereof will be omitted.

### (First Embodiment)

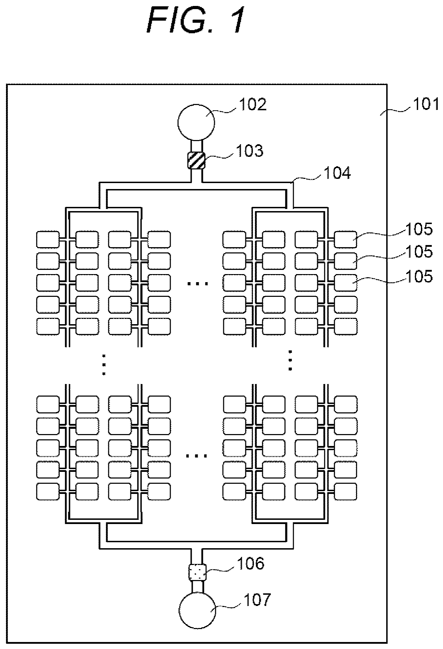

A sample solution separation device 101 according to a first embodiment will be described with reference to FIGS. 1 to 4. FIG. 1 is a configuration diagram of the sample solution separation device 101 according to the first embodiment. The sample solution separation device 101 is a device that separates a sample solution into a plurality of microchambers 105 and stores the sample solution therein. The sample solution separation device 101 includes a first opening 102, a valve 103, a flow path 104, a plurality of microchambers 105, a solid phase 106, and a second opening 107.

The flow path 104 is connected to the first opening 102 via the valve 103. The plurality of microchambers 105 is connected to the flow path 104. The flow path 104 is connected to the second opening 107 via the solid phase 106. That is, the flow path 104 connects the first opening 102, the plurality of microchambers 105, and the second opening 107.

The first opening 102 is an inlet for introducing the sample solution into the flow path 104.

The valve 103 is provided between the first opening 102 and the plurality of microchambers 105.

The second opening 107 is an inlet for introducing, to the flow path 104, a separation liquid for separation between the plurality of microchambers 105 storing the sample solution. The second opening 107 is provided on the opposite side of the first opening 102 such that the plurality of microchambers 105 are sandwiched between the first and second openings.

The solid phase 106 is provided between the second opening 107 and the plurality of microchambers 105. The solid phase 106 has air permeability, water repellency with respect to the sample solution, and permeability with respect to the separation liquid.

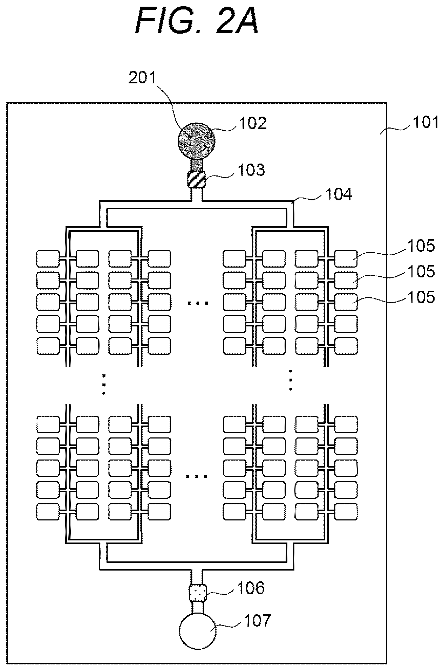

FIGS. 2A to 2D are diagrams showing how a sample solution 201 and a separation liquid 202 are to be introduced into the sample solution separation device 101 according to the first embodiment.

First, the sample solution 201 is introduced into the first opening 102. When the sample solution 201 is introduced into the first opening 102, the valve 103 is closed (FIG. 2A). After the valve 103 is closed, the air inside the flow path 104 and the microchambers 105 is degassed from the second opening 107 through the solid phase 106. That is, the flow path 104 and the microchambers 105 are placed in a vacuum state.

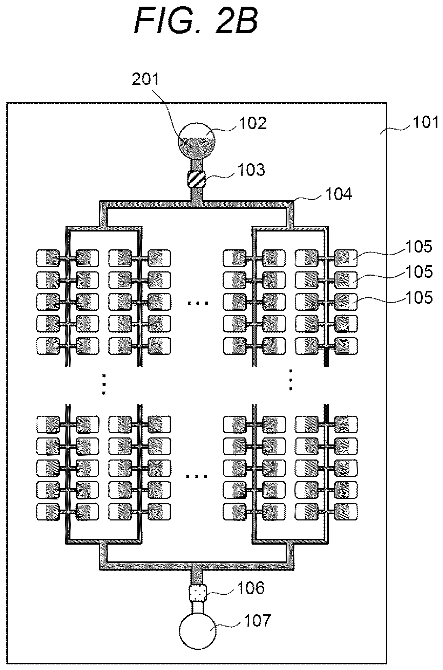

By placing the flow path 104 and the microchambers 105 in a vacuum state and opening the valve 103, the sample solution 201 is drawn into the flow path 104 and the microchambers 105 (FIG. 2B).

Here, the solid phase 106 is a solid having air permeability, hydrophobicity, and lipophilicity. The air permeability of the solid phase 106 allows air to pass therethrough, and as described above, the flow path 104 and the microchambers 105 can be placed in a vacuum state. In addition, due to the hydrophobicity of the solid phase 106, an aqueous solution such as the sample solution 201 can be made water-repellent, and the sample solution 201 can be prevented from passing through.

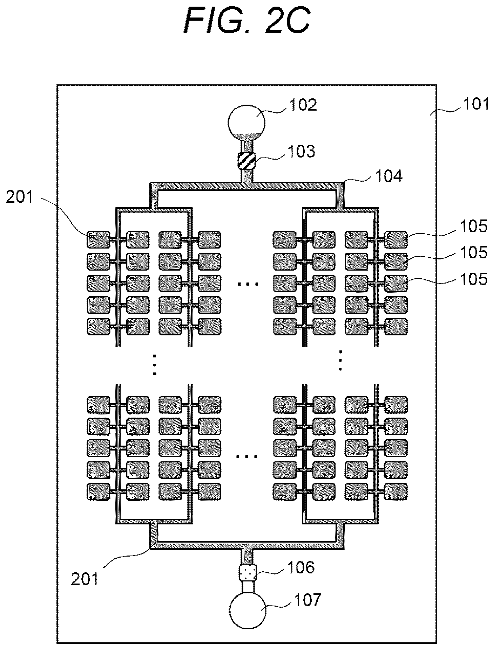

Therefore, the sample solution 201 drawn into the flow path 104 stops when reaching the solid phase 106. Because the interior of the microchambers 105 is in a vacuum state, the sample solution 201 is continuously drawn. The interior of the microchambers 105 is then filled with the sample solution 201 (FIG. 2C).

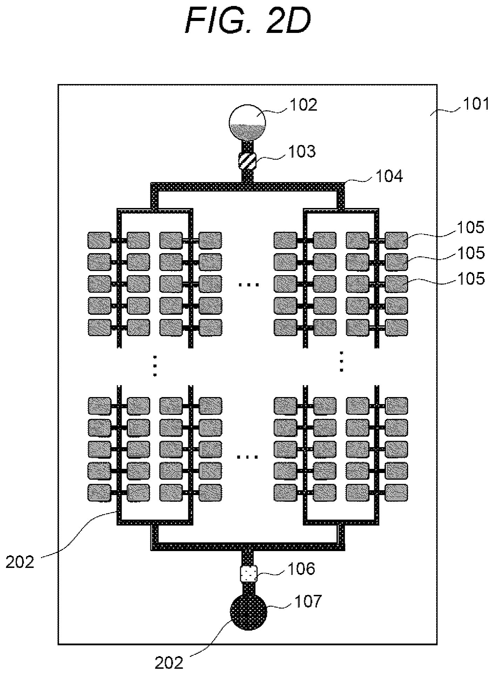

As the separation liquid 202 (see FIG. 2D) for separation between the plurality of microchambers 105 storing the sample solution 201, a separation liquid 202 having the physical property of being immiscible with the sample solution 201 (for example, oil) is used. Because the solid phase 106 has lipophilicity, the separation liquid 202 can pass therethrough.

Next, the sample solution 201 in the flow path 104 is replaced with the separation liquid 202. The separation liquid 202 is introduced from the second opening 107. Because the solid phase 106 has lipophilicity and has a property of allowing the separation liquid 202 to pass therethrough, the separation liquid 202 is introduced into the flow path 104 from the second opening 107 through the solid phase 106. The sample solution 201 in the flow path 104 is pushed back toward the first opening 102, and the flow path 104 is filled with the separation liquid 202 (FIG. 2D). At this time, because the sample solution 201 remains in the microchambers 105, the sample solution 201 in the plurality of microchambers 105 is separated by the separation liquid 202 in the flow path 104.

As described above, by using the solid phase 106 having air permeability, hydrophobicity, and lipophilicity, the air in the flow path 104 and the microchambers 105 can be removed, and the sample solution 201 can be stopped at the position of the solid phase 106. In the solid phase 106, the separation liquid 202 is introduced into the flow path 104, and the sample solution 201 can be divided and encapsulated in the microchambers 105.

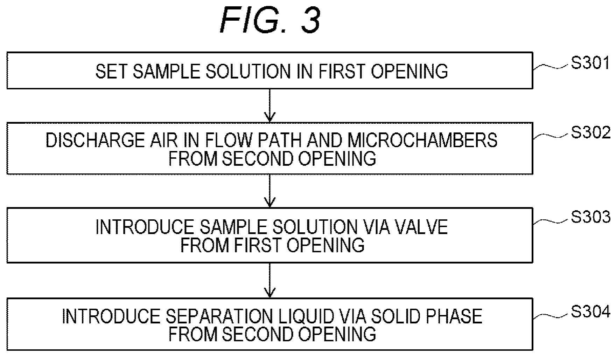

FIG. 3 is a flowchart for when the sample solution 201 and the separation liquid 202 are introduced into the sample solution separation device 101 according to the first embodiment, and when the sample solution 201 is separated into the microchambers 105.

First, the sample solution 201 is placed in the first opening 102 (S301). At this time, the valve 103 closes the flow path 104.

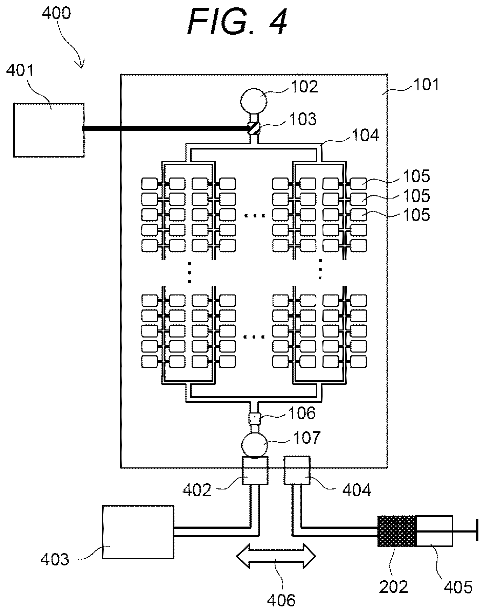

Thereafter, a pump 403 (see FIG. 4) connected to the second opening 107 is driven, and thus the air in the flow path 104 and the plurality of microchambers 105 is discharged through the solid phase 106 and the second opening 107 (S302). As a result, the flow path 104 and the plurality of microchambers 105 are placed in a vacuum state.

After the flow path 104 and the plurality of microchambers 105 have been placed in the vacuum state, the valve 103 opens the flow path 104, and thus the sample solution 201 is introduced into the flow path 104 and the plurality of microchambers 105 from the first opening 102 via the valve 103 (S303).

Thereafter, a pump 405 (see FIG. 4) connected to the second opening 107 is driven, and thus the separation liquid 202 is introduced into the flow path 104 through the second opening 107 and the solid phase 106 (S304). The sample solution 201 in the flow path 104 is pushed back in the direction of the first opening 102 by the separation liquid 202, the sample solution 201 inside the flow path 104 is replaced with the separation liquid 202, and thus separated between the plurality of microchambers 105 by the separation liquid 202. After the separation liquid 202 is introduced into the flow path 104, the valve 103 may be closed so that the sample solution 201 in the plurality of microchambers 105 and the separation liquid 202 in the flow path 104 do not move.

FIG. 4 is a configuration diagram of a sample solution separation system 400 in which the sample solution 201 is separated and introduced into the sample solution separation device 101 according to the first embodiment. The sample solution separation system 400 includes a valve control mechanism 401, an opening contact portion 402, the pump 403, an opening contact portion 404, the pump 405, a movement mechanism 406, and a sample solution separation device 101.

The valve control mechanism 401 (valve control means) controls the opening and closing of the valve 103. When the sample solution 201 is introduced into the plurality of microchambers 105, the valve control mechanism 401 changes the valve 103 from a closed state to an open state.

The opening contact portion 402 is connected to the second opening 107 of the sample solution separation device 101. In addition, the opening contact portion 402 is connected to the pump 403 that evacuates the flow path 104 and the plurality of microchambers 105. When the flow path 104 and the plurality of microchambers 105 are evacuated, the movement mechanism 406 moves the opening contact portion 402 to connect same to the second opening 107.

The pump 403 evacuates the flow path 104 and the plurality of microchambers 105 by degassing the air in the flow path 104 and the plurality of microchambers 105 from the second opening 107 through the solid phase 106.

The opening contact portion 404 is connected to the second opening 107 of the sample solution separation device 101. The opening contact portion 404 is connected to the pump 405 that introduces the separation liquid 202 into the flow path 104.

The movement mechanism 406 (switching means) connects the opening contact portion 402 to the second opening 107 when degassing the air from the flow path 104 and the plurality of microchambers 105. When the separation liquid 202 is introduced to the flow path 104, the movement mechanism 406 moves the opening contact portion 404 and connects the opening contact portion to the second opening 107. That is, the movement mechanism 406 switches the connection destination of the second opening 107 to the pump 403 or the pump 405.

The pump 405 (separation liquid introduction means) introduces the separation liquid 202 into the flow path 104 from the second opening 107 through the solid phase 106.

As the pump 403 for performing evacuation, a pump capable of reducing the pressure from atmospheric pressure to about 5 kPa to 0.01 kPa, for example, is used. A higher degree of vacuum allows more air to escape from the microchambers 105 and allows more sample solution 201 to enter therein.

As the pump 405 for introducing the separation liquid 202, for example, a syringe pump is used. The syringe pump pushes out the separation liquid 202 installed in advance and introduces the separation liquid 202 into the flow path 104. Note that the pump 405 may aspirate the separation liquid 202 in a container which is pre-installed and push out the aspirated separation liquid 202 to the flow path 104. The pump 405 may perform pressurization to introduce the separation liquid 202 into the flow path, and may be, for example, a metering discharge pump, a diaphragm pump, a rotary pump, or the like. By pressurizing the separation liquid 202 when the separation liquid 202 is introduced into the flow path 104, the separation liquid 202 can be pushed in even in a state where there is resistance of the solid phase 106 or flow path resistance.

Specifically, a porous membrane is used as the solid phase 106. For example, PTFE (polytetrafluoroethylene) is used as the solid phase 106. The solid phase 106 is porous and has air permeability so that air can pass therethrough, has water repellency so that the sample solution 201 can be stopped, and has lipophilicity so that the separation liquid 202 can pass therethrough. As the solid phase 106, a fluorine-based water-repellent film, a silicon-based water-repellent film, a polymer-based water-repellent film, or a nanoporous film, such as PFA (perfluoroalkoxyalkylene), FEP (fluoroethylene propylene), or ETFE (ethylene tetrafluoroethylene), may be used. The solid phase 106 may not be a membrane, or may be one in which beads having hydrophobicity are packed in the flow path 104. The beads are hydrophobic and therefore can stop the sample solution 201 by repelling the sample solution 201. As the porous membrane, a hydrophobic membrane having water-repellency at a contact angle with water of 80 degrees or more, for example, may be used. The solid phase 106 is made of a material that is lipophilic and allows the separation liquid 202 to permeate therethrough so that the separation liquid 202 can pass therethrough.

Conventionally, for example, as in the method disclosed in NPL 1, a valve has been used instead of the solid phase 106 described above. In NPL 1, the sample solution can be stopped when the valve is closed, and the air can be removed or the separation liquid can be introduced when the valve is opened. However, for example, in a case where the valve is a pinch valve that crushes the tube, a portion from the flow path to the tube and a volume through which the sample solution passes through the tube are required. The sample solution remaining in the tube cannot be used for measurement, and thus becomes dead volume. This dead volume reduces measurable target DNA, leading to a decrease in detection sensitivity. Meanwhile, by using the solid phase 106 according to the first embodiment, the solid phase 106 can be arranged near the microchambers 105, and the dead volume of the sample solution 201 can be reduced.

As described above, by using the solid phase 106 according to the first embodiment, the solid phase 106 can be mounted on the flow path 104, and hence dead volume such as a valve can be suppressed. Although the sample solution 201 remaining in the flow path 104 may become dead volume, the volume of the sample solution 201 remaining in the flow path 104 can be minimized and the dead volume of the sample solution 201 can be reduced by designing and machining the flow path 104 to have a small cross-sectional area.

In the first embodiment, the valve 103 is used before the sample solution 201 is introduced into the flow path 104. The valve 103 is in a closed state during evacuation, and is in an open state when the sample solution 201 is introduced. As the valve 103, a one-time valve that is opened only once when the sample solution 201 is introduced into the flow path 104 can also be used. Specifically, for example, a resin film is used as a valve, the valve is normally closed, and the valve is placed in an open state by breaking the film when the sample solution 201 is to be introduced. This configuration makes it possible to provide a low-cost and small-sized device. Note that, as the valve 103, an electromagnetic valve or a pinch valve for crushing a tube may be used.

As the separation liquid 202, a liquid having the physical property of being immiscible with the sample solution 201 is used. Specifically, for example, oil such as a silicone oil or mineral oil, paraffin wax, a photocurable resin, or the like can be used as the separation liquid 202.

The separation liquid 202 may be a photocurable resin. In a case where the sample solution 201 is to be separated using a photocurable resin, the photocurable resin is introduced into the flow path 104 in a liquid state. Thereafter, the photocurable resin is solidified by light irradiation such as ultraviolet rays, and can be separated and sealed in the plurality of microchambers 105 in which the sample solution 201 is stored. In addition, a plurality of kinds of separation liquids may be used instead of one kind of separation liquid. Specifically, for example, the photocurable resin and the oil are introduced in this order from the second opening 107. The photocurable resin reaches the vicinity of the valve 103, and the flow path 104 is filled with oil. Thereafter, by curing the photocurable resin, the end of the flow path 104 can be sealed with the photocurable resin, and the microchambers 105 can be separated from each other with oil.

The sample solution 201 is, for example, a solution containing the DNA to be measured, a polymerase, a buffer, a primer, and a probe. The amount of the solution is adjusted to about 10 µL to about 50 µL.

In the first embodiment, the dead volume of the sample solution 201 can be reduced as compared with a configuration in which valves are used on both sides of the flow path as per the related art. Therefore, most of the adjusted sample solution 201 can be used for measurement, and highly sensitive detection can be performed.

Note that when the sample solution 201 is introduced into the first opening 102, another liquid (separation liquid) such as oil may be introduced continuously with the sample solution 201. Thus, the sample solution 201 can be pushed into the microchambers 105 with the oil. As a result, because the interior of the flow path 104 can be filled with the separation liquid such as oil, the dead volume of the sample solution 201 in the flow path 104 can be further reduced. In addition, when the separation liquid 202 is introduced, the liquid pushed back to the first opening 102 is oil (the separation liquid) and plays the role of a lid, and therefore it is possible to prevent nucleic acids in the sample solution 201 from diffusing into the atmosphere.

Thus, the sample solution separation device 101 with which the sample solution 201 is separately introduced into multiple microchambers 105 is subjected to a PCR thermal cycle. Due to the thermal cycle, the target DNA is amplified by the PCR in the plurality of microchambers 105. The thermal cycle is set, for example, at a high temperature of 95°C for 20 seconds and at a low temperature of 60°C for 40 seconds, and the double helix is denatured at the high temperature, and annealed and extended at the low temperature, and the PCR thus amplified. The target DNA separated and introduced into the plurality of microchambers 105 can be detected by measuring the fluorescence of the amplification product.

Note that the volume of the microchambers 105 is, for example, about several pL to several nL, and the number of the microchambers 105 is about several thousand to several million. In the digital PCR, by dividing the target DNA into multiple microchambers 105, the amount of background DNA can be reduced, and the target DNA can be detected with high sensitivity.

Note that, in the present embodiment, the opening contact portion 402 and the opening contact portion 404 are switched using the movement mechanism 406 (switching means), but the flow path may be switched using an electromagnetic valve or the like.

The microchambers 105 and the flow path 104 are connected by a thin connection flow path which branches from the main flow path. The thin connection flow path affords robust separation between the plurality of microchambers 105. When the separation liquid 202 is introduced into the flow path 104, the sample solution 201 introduced into the microchambers 105 flows through the main flow path. The connection flow path has a smaller flow path cross-sectional area than that of the main flow path. In addition, because the microchambers 105 ahead of the connection flow path are filled with the sample solution 201, the separation liquid 202 does not enter the microchambers 105 from the connection flow path but flows through the main flow path. Additionally, because interfacial tension acts between the sample solution 201 and the separation liquid 202, the sample solution 201 takes on the form of droplets in the microchambers 105, and the plurality of microchambers 105 are separated by the separation liquid 202. As described above, by connecting the connection flow path having a cross-sectional area smaller than that of the main flow path to the plurality of microchambers 105, the separation liquid 202 does not enter the microchambers 105, but passes through the flow path 104, thus enabling the sample solution 201 to be separated.

### (Second Embodiment)

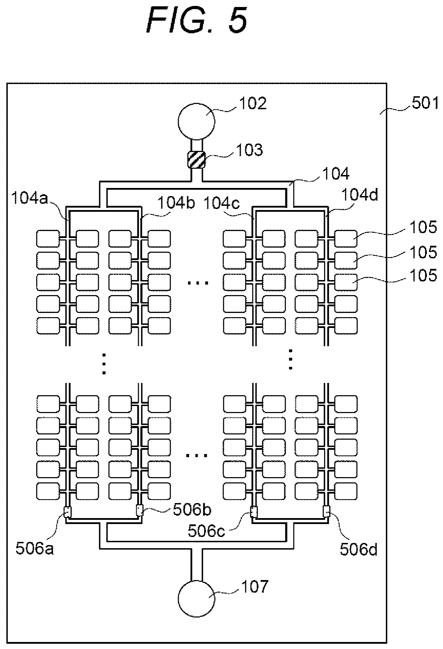

In the first embodiment, an example in which the solid phase 106 is disposed at one position of the flow path 104 has been described, but the present invention is not limited to this example. FIG. 5 is a configuration diagram of a sample solution separation device 501 according to the second embodiment. The sample solution separation device 501 includes a first opening 102, a valve 103, a flow path 104, a plurality of microchambers 105, a plurality of solid phases 506a to 506d, and a second opening 107. Note that the same description as in the first embodiment will be omitted as appropriate. The flow path 104 is branched to connect to multiple microchambers 105. The plurality of branch flow paths 104a to 104d branching from the main flow path are connected to multiple microchambers 105 and then merged again and connected to the second opening 107. The solid phases 501a to 501d through which air is allowed to pass, the sample solution 201 is stopped, and the separation liquid 202 is allowed to pass can be installed at a plurality of positions between the microchambers 105 and the second opening 107. In the example of FIG. 5, the solid phases 501a to 501d are provided in each of the plurality of branch flow paths 104a to 104d of the flow path 104. The number of the plurality of branch flow paths 104a to 104d and the number of the solid phases 501a to 501d are not limited to 4, and may be 2 to 3 or 5 or more.

As in the second embodiment, by arranging the solid phases 501a to 501d closer to the microchambers 105 as compared with the first embodiment, the sample solution 201 remaining in the flow path 104 can be reduced, and the dead volume of the sample solution 201 can be reduced.

### (Third Embodiment)

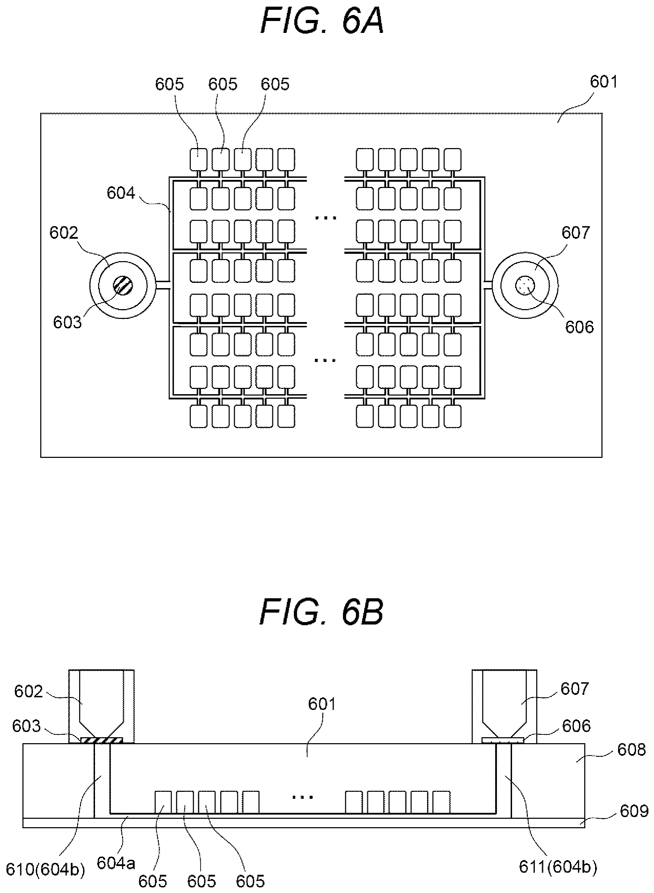

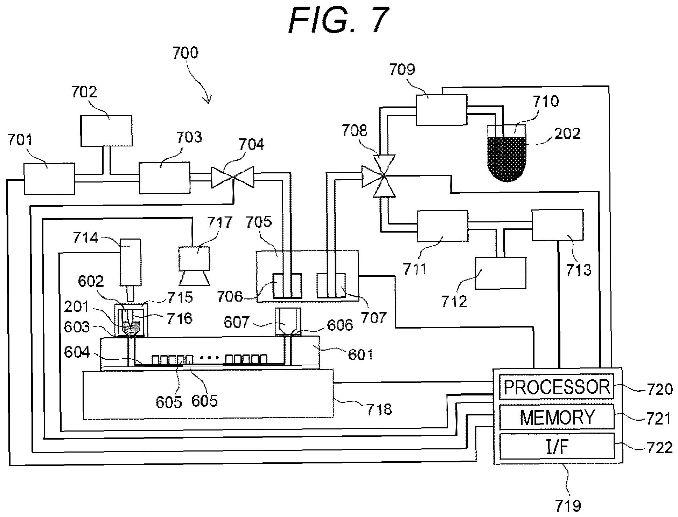

The sample solution separation device 601 according to the third embodiment will be described with reference to FIGS. 6A, 6B, and 7. FIG. 6A is a top view of the sample solution separation device 601 according to the third embodiment, and FIG. 6B is a lateral view of the sample solution separation device 601 according to the third embodiment. As shown in FIGS. 6A and 6B, the sample solution separation device 601 includes a first opening 602, a valve 603, a flow path 604, a plurality of microchambers 605, a solid phase 606, and a second opening 607. Note that the same description as in the first and second embodiments will be omitted as appropriate. The flow path 604 and the microchambers 605 are processed into a substrate 608. Through-holes 610 and 611 penetrating the substrate 608 are formed at one end and the other end of the flow path 604. The through-hole 610 is connected to the first opening 602 via the valve 603, and the through-hole 611 is connected to the second opening 607 through the solid phase 606. In addition, the side of the substrate 608 whereon the plurality of microchambers 605 and the flow path 604 are processed is sealed with a film 609.

That is, the flow path 604 of the sample solution separation device 601 according to the third embodiment has a horizontal flow path 604a to which the plurality of microchambers 605 are connected and extending in the horizontal direction, and a vertical flow path 604b (through-holes 610 and 611) connected to the horizontal flow path 604a and extending in the vertical direction. The solid phase 606 is provided at the upper end of the vertical flow path 604b (the through-hole 611).

A resin is used as the material for the substrate 608 or the film 609, but the present invention is not limited to a resin. For example, the material of the substrate 608 or the film 609 may be a COP (cycloolefin polymer) having low autofluorescence or a COC (cycloolefin copolymer). The material of the substrate 608 or the film 609 may be a polycarbonate, polypropylene, or PMMA (methacrylic resin), or the like. Further, a part of the substrate 608 or the film 609 may be a metal such as aluminum having high thermal conductivity, or a material such as carbon which is capable of suppressing light reflection.

The valve 603 is a normally closed seal disposed so as to close the through-hole 610, and is a one-time valve that changes from a closed state to an open state when a hole is formed. A first opening 602 having a volume capable of receiving the sample solution 201 is disposed in the upper portion of the valve 603. The solid phase 606 has a film shape and is disposed so as to close the through-hole 611. The second opening 607 having a volume capable of receiving the separation liquid 202 is disposed in the upper portion of the solid phase 606. With such a configuration, the valve 603 and the solid phase 606 can be easily arranged on the substrate 608. In addition, because the solid phase 606 can be disposed near the flow path 604, the dead volume of the sample solution 291 can be reduced.

FIG. 7 is a configuration diagram of a sample solution separation system 700 in which the sample solution 201 is separated and introduced into the sample solution separation device 601 according to the third embodiment. The sample solution separation system 700 includes a vacuum pump 701, a pressure sensor 702, a filter 703, an electromagnetic valve 704, a movement mechanism 705, an opening contact unit 706, an opening contact unit 707, an electromagnetic valve 708, a liquid pump 709, a container 710, a filter 711, a pressure sensor 712, a pressurizing pump 713, a valve control mechanism 714, an opening lid 715, a light source 717, a temperature controller 718, a controller 719, and a sample solution separation device 601.

The sample solution 201 is filled in advance in the first opening 602 and installed so as to be in contact with the upper portion of the valve 603. At this time, the valve 603 is in a closed state. The first opening 602 is provided with the opening lid 715. On the inner side of the upper surface of the opening lid 715, a pointed component 716 having a pointed tip and capable of breaking the seal (valve 603) is installed. The upper surface of the opening lid 715 is made of a stretchable material, for example, an elastomer or rubber.

The second opening 607 of the sample solution separation device 601 and the opening contact portion 706 are connected by controlling the movement mechanism 705 (switching means). Further, the electromagnetic valve 704 connects or disconnects the vacuum pump 701 and the opening contact portion 706. The vacuum pump 701 evacuates the flow path 604 and the plurality of microchambers 605 of the sample solution separation device 601. After the flow path 604 and the plurality of microchambers 605 are sufficiently depressurized, the valve control mechanism 714 (valve control means) pushes the upper portion of the opening lid 715, whereby the pointed component 716 breaks the valve 603. As a result, the valve 603 is placed in an open state. Here, as described above, the valve 603 is formed by a thin-film seal made of aluminum, resin, or the like, and can be broken by the pointed component 716. Because the flow path 604 and the plurality of microchambers 605 are depressurized, the sample solution 201 is drawn into the flow path 604 and the plurality of microchambers 605, and the flow path 604 and the plurality of microchambers 605 are filled with the sample solution 201. Because the solid phase 606 has water repellency, the sample solution 201 passing through the flow path 604 stops at the solid phase 606. In addition, because the solid phase 606 has air permeability, the interior of the flow path 604 and the microchambers 605 can be placed under a negative pressure by the vacuum pump 701 until the tip of the sample solution 201 reaches the solid phase 606, and the degree of vacuum can be increased.

The electromagnetic valve 704 is used to seal the flow path 604, connect the vacuum pump 701 and the opening contact portion 706, and provide exposure to the atmosphere. By opening the second opening 607 to the atmosphere, the flow path 604 of the sample solution separation device 601 is exposed to atmospheric pressure. Thereafter, the movement mechanism 705 connects the opening contact portion 707 and the second opening 607. The electromagnetic valve 708 connects the opening contact portion 707 and the liquid pump 709. The liquid pump 709 aspirates the separation liquid 202, which is pre-installed in the container 710, and discharges the separation liquid toward the opening contact portion 707. The separation liquid 202 is introduced into the flow path 604 through the second opening 607 and the solid phase 606.

The electromagnetic valve 708 switches the connection destination to the second opening 607 from the liquid pump 709 to the pressurizing pump 713. The pressurizing pump 713 pressurizes the separation liquid 202 and pushes same into the flow path 604. The pressurizing pump 713 introduces the separation liquid 202 into the flow path 604 by providing pressurization at a pressure exceeding the flow path resistance of the flow path 604 or the resistance during passage through the solid phase 606. The pressure at the time of pressurization is determined by the separation liquid 202, the size of the flow path 604, the properties of the solid phase 606, and the like, and is, for example, about 10 kPa to 200 kPa. The sample solution 201 in the flow path 604 is pushed back toward the first opening 602, and the flow path 604 is filled with the separation liquid 202. Thus, the sample solution 201 remains in each microchamber 605 and is separated by the separation liquid 202. In addition, by pressurizing and introducing the separation liquid 202 into the flow path 604, the expansion of bubbles remaining for whatever reason can be suppressed, and the separation liquid 202 can be introduced into the flow path 604.

The separation liquid 202 may also be a photocurable resin. By using a photocurable resin that is immiscible with the sample solution 201, the sample solution 201 can be separated into the microchambers 605. In this case, after the photocurable resin in the liquid state is introduced into the flow path 604, the photocurable resin is cured using the light source 717. Thus, the sample solution 201 can be sealed in the microchambers 605.

When the sample solution 201 or the separation liquid 202 is introduced, the temperature of the sample solution separation device 601 can be controlled using the temperature controller 718. In general, the viscosity of the sample solution 201 or the separation liquid 202 decreases as the temperature increases. Therefore, the viscosity of the sample solution 201 and the separation liquid 202 decreases as a result of being heated rather than being at room temperature, and the sample solution 201 and the separation liquid 202 easily enter the flow path 604 and the microchambers 605. As a result, the introduction time of the solution (the sample solution 201 and the separation liquid 202) can be shortened, and the sample solution 201 can be placed in all the microchambers 605 in a robust manner. In the control by the temperature controller 718, for example, heating to about 35°C to 70°C is preferable.

The pressure during evacuation by the vacuum pump 701 is monitored and controlled by the pressure sensor 702. The pressure during pressurization by the pressurizing pump 713 is monitored and controlled by the pressure sensor 712. In addition, the filter 703 and the filter 711 prevent dust or the like from being mixed into the sample solution 201 and the separation liquid 202.

The control of the series of operations described above is performed by the controller 719. The controller 719 includes a processor 720, a memory 721, and an interface 722. The processor 720 executes various programs in the memory 721 and outputs control signals for controlling the above-described operations via the interface 722.

Note that the valve control mechanism 714 is, for example, a solenoid, and moves when a current flows, thereby opening the valve 603. The movement mechanism 705 is, for example, a two-axis motor drive mechanism in the horizontal direction and the vertical direction.

By using the solid phase 606 having air permeability, hydrophobicity, and lipophilicity, it is possible to control evacuation of the flow path 604, stoppage of the sample solution 201, and passage of the separation liquid 202 without using a valve. In addition, the solid phase 606 can be arranged near the microchambers 605, and the sample solution 201 can be divided and placed in the microchambers 605 without wasting the sample solution 201.

Further, a valve having a simple configuration can be implemented by using, as the valve 603, a one-time valve having a configuration in which a hole is formed in a thin film. Because the sample solution 201 is in contact with the valve 603, the valve needs to be disposable in order to prevent carryover, and thus a one-time valve is useful. Furthermore, because the valve 603 is a valve having a simple configuration, the valve can be provided at low cost. In addition, the microchambers 605 can be sealed by using a photocurable resin as the separation liquid 202, placing the photocurable resin in the flow path 604 in a liquid state, and then solidifying the photocurable resin. This process eliminates the need to close the first opening 602 and second opening 607, and thus the configuration can be simplified.

Note that the valve 603 may be pierced with a pipette tip. The sample solution separation device 601 according to the third embodiment can also be applied to an automated system connected to a sample pretreatment for the extraction of nucleic acids, the mixing of reagents, pipetting, and the like. For example, a sample extracted in a blood collection tube is centrifuged to extract plasma, and the plasma is purified to extract nucleic acids in the plasma. The nucleic acids are mixed with a reagent, and nucleic acids of interest are detected using digital PCR. At this time, the sample solution 201 is injected into the first opening 602 using a pipetting system that aspirates and discharges the mixed sample solution by means of a pipette tip. At this time, the valve may be broken at the tip of the pipette tip, and the sample solution 201 may be introduced into the flow path.

In addition, the dead volume of the sample solution 201 can be further reduced by introducing the oil (separation liquid) continuously with the sample solution 201 from the first opening 602. Specifically, because the oil (separation liquid) continuously enters the flow path 604 after the sample solution 201 is introduced into the microchambers 605 by the negative pressure, the sample solution 201 remaining in the flow path 604 can be reduced. By placing the separation liquid 202 in the direction opposite to the sample solution 201 from the second opening 607 in this state, the sample solution 201 in the flow path 604 is completely replaced with the separation liquid 202, and the plurality of microchambers 605 can be separated. In addition, the oil (separation liquid) continuously introduced with the sample solution 201 serves as a lid to prevent the nucleic acids in the sample solution 201 from becoming an aerosol and being released into the atmosphere. It is thus possible to further reduce the dead volume of the sample solution 201 as compared with the case where only the sample solution 201 is introduced from the first opening 602.

Note that, in the present embodiment, the separation liquid 202 in the container 710 is placed in the flow path 604 through the second opening 607, but the present invention is not limited to this configuration. For example, the separation liquid 202 may be filled in the sample solution separation device 601 in advance, and the separation liquid 202 filled in the sample solution separation device 601 may be introduced into the flow path 604.

### (Fourth embodiment)

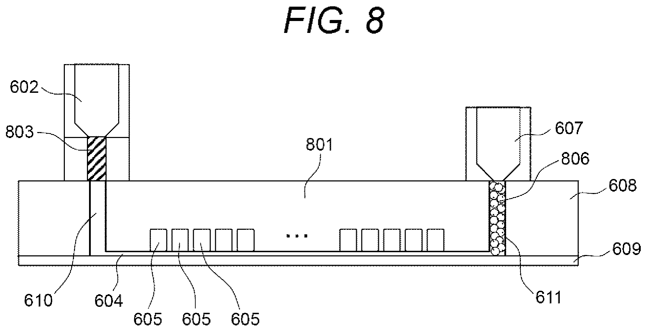

In the third embodiment described above, the thin-film solid phase 606 is provided at the upper end of the through-hole 611 of the substrate 608, but the present invention is not limited to this configuration. FIG. 8 is a configuration diagram of a sample solution separation device 801 according to the fourth embodiment. A solid phase 806 of the sample solution separation device 801 according to the fourth embodiment is a plurality of fine particles having air permeability, hydrophobicity, and lipophilicity. The plurality of fine particles are packed in the through-hole 611 (vertical flow path) to form the solid phase 806. The fine particles are, for example, beads having a size of 0.1 µm to 10 µm. The fine particles are more easily introduced into the flow path 604 than a film. In addition, because air can pass between the fine particles, evacuation can be performed. Further, the fine particles have water repellency, and therefore the sample solution 201 can be stopped. Furthermore, because the fine particles have lipophilicity, the fine particles can pass through the separation liquid 202.

In the third embodiment, the valve 603 is a one-time valve, but the present invention is not limited thereto. For example, the valve 803 of the sample solution separation device 800 according to the fourth embodiment may be a stretchable tube. The tube is extended to connect with the through-hole 610 of the substrate 608 and forms the valve 803. For example, the valve 803 is a silicone tube. The silicone tube is compressed to form a pinch valve that stops flow in the tube. By using the pinch valve, opening and closing can be performed a plurality of times. The valve 803 is closed at the time of evacuation, and the valve 803 is opened when the sample solution 201 is added. After introduction of the separation liquid 202, the valve 803 is closed to seal the sample solution 201 and the separation liquid 202.

### (Fifth Embodiment)

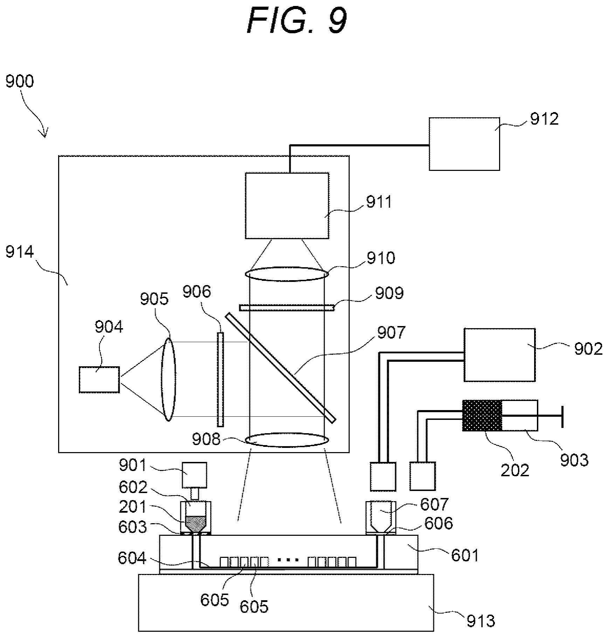

The sample solution separation device of the present invention can be used for digital PCR. FIG. 9 is a configuration diagram of a digital PCR system 900 according to a fifth embodiment. The digital PCR system 900 (the sample solution separation system) includes a sample solution separation device 601, a valve control mechanism 901, a pump 902, a liquid pump 903, a temperature controller 913, an optical system 914 (measurement means), and an analysis unit 912. The optical system 914 includes a light source 904, a lens 905, a lens 908, a lens 910, a bandpass filter 906, a bandpass filter 909, a dichroic mirror 907, and a CMOS sensor 911.

The sample solution 201 is placed in the sample solution separation device 601 through the first opening 602. By driving the pump 902, the air in the flow path 604 and the microchambers 605 is degassed through the second opening 607 to establish a negative pressure. The valve control mechanism 901 opens the valve 603, and the sample solution 201 is introduced into the flow path 604 and the microchambers 605. Thereafter, by driving the liquid pump 903, the separation liquid 202 is introduced into the flow path 604 via the second opening 607. Thus, the sample solution 201 can be separately introduced into the plurality of microchambers 605.

Here, the sample solution 201 is a PCR reaction solution containing the DNA to be detected, a polymerase, a buffer, a primer, and a probe. A device in which the sample solution 201, which is a PCR reaction solution, is separately placed in multiple microchambers 605, is subjected to thermal cycle processing. In the thermal cycle processing, the temperature controller 913 is controlled to a temperature zone in which denaturation, annealing, and extension are performed. The temperature controller 913 is configured from a heater, a Peltier element, or the like, but the present invention is not limited thereto. Due to the thermal cycle processing, in a case where the DNA to be measured is present in the microchambers 605, the target DNA is amplified.

The optical system 914 (measurement means) measures target nucleic acids in the sample solution 201 subjected to PCR amplification. The light emitted from the light source 904 is collimated by the lens 905, and light of a predetermined wavelength is transmitted by the bandpass filter 906 and reflected by the dichroic mirror 907 before passing through the lens 908 to irradiate the sample solution separation device 601. The fluorescence from the microchambers 605 of the sample solution separation device 601 passes through the lens 908, passes through the dichroic mirror 907, the bandpass filter 909, and the lens 910, and is imaged by the CMOS sensor 911. The analysis unit 912 performs analysis on the basis of an image captured by the CMOS sensor 911, and detects target DNA in each microchamber.

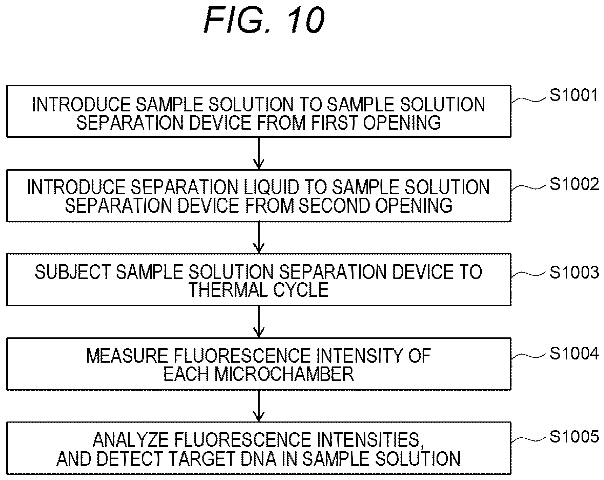

FIG. 10 is an operation flowchart of a digital PCR system 900 according to the fifth embodiment.

The valve control mechanism 901 opens the valve 603 to introduce the sample solution 201 placed in the first opening 602 into the sample solution separation device 601 (S1001).

The liquid pump 903 introduces the separation liquid 202 into the sample solution separation device 601 through the second opening 607 (S1002). As a result, the sample solution 201 is separately introduced into the plurality of microchambers 605.

Next, the temperature controller 913 applies the sample solution separation device 601 to a thermal cycle (S1003).

The optical system 914 irradiates the sample solution separation device 601 with light and measures the fluorescence intensity of each microchamber 605 (S 1004).

An analysis unit 915 then detects the target DNA in the sample solution 201 by analyzing the fluorescence intensity measured by the optical system 914 (S1005).

When the sample solution 201 or the separation liquid 202 is to be introduced, the sample solution 201 or the separation liquid 202 may be heated. The viscosity of the sample solution 201 or the separation liquid 202 is changed, thereby facilitating introduction into the flow path 604. In addition, the time for introducing the sample solution 201 or the separation liquid 202 can be shortened. The temperature controller that heats the sample solution 201 and the separation liquid 202 may be the temperature controller 913 that implements a thermal cycle, or may be a temperature controller different from the temperature controller 913.

As the PCR reaction solution, asymmetric PCR may be performed by changing the concentrations of the primer on the forward side and the primer on the reverse side so that a large amount of one single-stranded DNA of the amplification products is amplified. Thus, single-stranded DNA is detected using molecular beacons. In addition, the temperature of the sample solution separation device is controlled at the time of fluorescence measurement, and melting curve analysis is performed. The fluorescence measurement is performed in a plurality of colors by using a plurality of filters. By specifying target DNA using the color of fluorescence, the fluorescence intensity, and the melting temperature of each microchamber, highly multiplexed and highly sensitive measurement can be implemented.

### (Sample Solution Separation Method)

Here, a method for separating the sample solution 201 into a plurality of microchambers 105 will be described. The sample solution separation method includes:
preparing the sample solution separation device 101;
degassing air in the plurality of microchambers 105 and the flow path 104 via the solid phase 106;
opening the valve 103 and introducing the sample solution 201 from the first opening 102 into the plurality of microchambers 105 and the flow path 104; and
introducing the separation liquid 202 into the flow path 104 through the solid phase 106 from the second opening 107.

The sample solution separation device 101 to be prepared may be the sample solution separation device 501, 601, or 801.

Further, the sample solution separation method further includes:
performing a thermal cycle of PCR (polymerase chain reaction) on the sample solution separation device 101 with which the sample solution 201 is separated into the plurality of microchambers 105;
measuring fluorescence intensities of the plurality of microchambers 105; and
analyzing the fluorescence intensities and detecting target DNA in the sample solution 201.

Note that the introduction of the sample solution 201 from the first opening 102 into the plurality of microchambers 105 and the flow path 104 includes the introduction of the sample solution 201 and the oil (separation liquid) following the sample solution 201 from the first opening 102.

### (Modifications)

The present invention is not limited to the above-described embodiments, and includes various modifications. For example, the above-described embodiments have been described in detail for easy understanding of the present invention, but the present invention is not necessarily limited to embodiments having all the described configurations. Further, part of the configuration of one embodiment can be replaced with the configuration of another embodiment, and the configuration of another embodiment can also be added to the configuration of the one embodiment. In addition, it is possible to add or eliminate other configurations to/from part of the configuration of each embodiment, or other configurations can be substituted for part of the configuration of each embodiment.

For example, in the above-described embodiments, the separation liquid is used to separate the plurality of microchambers 105, but the present invention is not limited thereto, and a separation gas may instead be used. In this case, the solid phase may have a physical property of allowing the separated gas to pass therethrough.

### Reference Signs List

101, 501, 601, 801 sample solution separation device
102 first opening
103 valve
104 flow path
105 microchamber
106 solid phase
107 second opening
201 sample solution
202 separation liquid
400, 700 sample solution separation system
401 valve control mechanism
402, 404 opening contact portion
403, 405 pump
406 movement mechanism
506a, 506b, 506c, 506d solid phase
602 first opening
603 valve
604 flow path
604a horizontal flow path
604b vertical flow path
605 microchamber
606 solid phase
607 second opening
608 substrate
609 film
610, 611 through-hole
701 vacuum pump
702, 712 pressure sensor
703, 711 filter
704, 708 electromagnetic valve
705 movement mechanism
706, 707 opening contact portion
709 liquid pump
710 container
713 pressurizing pump
714 valve control mechanism
715 opening lid
716 pointed component
717 light source
718 temperature controller
719 controller
720 processor
721 memory
722 interface
803 valve
806 solid phase
900 digital PCR system
901 valve control mechanism
902 pump
903 liquid pump
904 light source
905, 908, 910 lens
906, 909 bandpass filter
907 dichroic mirror
911 CMOS sensor
912 analysis unit
913 temperature controller
914 optical system

## Claims

1. A sample solution separation device for separating a sample solution, the device comprising:
a plurality of microchambers;
a flow path connecting the plurality of microchambers;
a first opening constituting an inlet through which the sample solution is introduced into the flow path;
a valve provided between the first opening and the plurality of microchambers;
a second opening provided on an opposite side to the first opening such that the plurality of microchambers are sandwiched between the first and second openings, the second opening being an inlet to a flow path of a separation liquid for separation between the plurality of microchambers in which the sample solution is stored; and
a solid phase provided between the second opening and the plurality of microchambers,
wherein the solid phase has air permeability, water repellency with respect to the sample solution, and permeability with respect to the separation liquid.

2. The sample solution separation device according to claim 1,
wherein the flow path includes a plurality of branch flow paths to which the microchambers are respectively connected, and
wherein the solid phase is provided in each of the plurality of branch flow paths.

3. The sample solution separation device according to claim 1,
wherein the flow path includes:
a horizontal flow path to which the plurality of microchambers are connected and which extends in a horizontal direction; and
a vertical flow path connected to the horizontal flow path and extending in a vertical direction,
wherein the solid phase is provided at an upper end of the vertical flow path.

4. The sample solution separation device according to claim 1,
wherein the flow path includes:
a horizontal flow path to which the plurality of microchambers are connected and which extends in a horizontal direction; and
a vertical flow path connected to the horizontal flow path and extending in a vertical direction,
wherein the solid phase is a plurality of fine particles provided in the vertical flow path.

5. The sample solution separation device according to claim 1,
wherein the valve is a one-time valve that is opened only once when the sample solution is to be introduced into the flow path.

6. The sample solution separation device according to claim 1,
wherein the solid phase is a porous membrane having water repellency to the sample solution and having permeability to the separation liquid.

7. The sample solution separation device according to claim 6,
wherein the material of the porous membrane is PTFE (polytetrafluoroethylene).

8. The sample solution separation device according to claim 1,
wherein the separation liquid is a photocurable resin, and
wherein the separation liquid is cured by light irradiation in the flow path.

9. The sample solution separation device according to claim 1,
wherein the separation liquid is an oil having the physical property of being immiscible with the sample solution.

10. A sample solution separation system, comprising:
a sample solution separation device that separates a sample solution and that includes a plurality of microchambers, a flow path connecting the plurality of microchambers, a first opening constituting an inlet through which the sample solution is introduced into the flow path, a valve provided between the first opening and the plurality of microchambers, a second opening provided on an opposite side to the first opening such that the plurality of microchambers are sandwiched between the first and second openings, the second opening being an inlet to a flow path of a separation liquid for separation between the plurality of microchambers in which the sample solution is stored, and a solid phase provided between the second opening and the plurality of microchambers, the solid phase having air permeability, water repellency with respect to the sample solution, and permeability with respect to the separation liquid, the sample solution separation system further comprising:
a pump that degasses the air in the plurality of microchambers and the flow path via the solid phase from the second opening;
valve control means for opening the valve; and
separation liquid introduction means for introducing the separation liquid from the second opening.

11. The sample solution separation system according to claim 10, further comprising:
switching means for switching a connection destination of the second opening to the pump or the separation liquid introduction means.

12. The sample solution separation system according to claim 10, further comprising:
a pressurizing pump that pressurizes the separation liquid and introduces the separation liquid into the flow path.

13. The sample solution separation system according to claim 10, further comprising:
measurement means for measuring nucleic acids of interest in the sample solution subjected to PCR (polymerase chain reaction) in the microchamber.

14. A sample solution separation method, comprising:
preparing a sample solution separation device for separating a sample solution and that includes a plurality of microchambers, a flow path connecting the plurality of microchambers, a first opening constituting an inlet through which the sample solution is introduced into the flow path, a valve provided between the first opening and the plurality of microchambers, a second opening provided on an opposite side to the first opening such that the plurality of microchambers are sandwiched between the first and second openings, the second opening being an inlet to a flow path of a separation liquid for separation between the plurality of microchambers in which the sample solution is stored, and a solid phase provided between the second opening and the plurality of microchambers, the solid phase having air permeability, water repellency with respect to the sample solution, and permeability with respect to the separation liquid;
degassing the air in the plurality of microchambers and the flow path via the solid phase;
opening the valve and introducing the sample solution from the first opening to the plurality of microchambers and the flow path; and
introducing the separation liquid from the second opening to the flow path via the solid phase.

15. The sample solution separation method according to claim 14, further comprising:
performing a thermal cycle of PCR (polymerase chain reaction) on the sample solution separation device with which the sample solution is separated into the plurality of microchambers;
measuring fluorescence intensities of the plurality of microchambers; and
analyzing the fluorescence intensities and detecting target DNA in the sample solution.

16. The sample solution separation method according to claim 14,
wherein introducing the sample solution into the plurality of microchambers and the flow path from the first opening includes introducing the sample solution and a separation liquid subsequent to the sample solution from the first opening.
